# FASCICULE DE BREVET EUROPEEN

(11) **EP 1 234 560 B1**
(45) Date de publication et mention de la délivrance du brevet: **15.12.2004**
(21) Numéro de dépôt: 02356033.7
(22) Date de dépôt: 20.02.2002
(51) Int. Cl.: A61F 5/01

(54) **Orthèse pour entorse externe de la cheville**
Orthese für ein extern verstauchtes Sprunggelenk
Orthosis for an externally sprained ankle

(30) Priorité: 21.02.2001 FR 0102331
(43) Date de publication de la demande: 28.08.2002
(73) Titulaire: GIBAUD, SOCIETE ANONYME, F-42000 Saint Etienne (FR)
(72) Inventeur: Pichon, Jean-Claude M., 42000 Saint Etienne (FR)
(74) Mandataire: Maureau, Philippe

(56) Documents cités:
- US-A- 4 646 726
- US-A- 4 966 134
- US-A- 5 199 941
- US-A- 5 445 603

## Description

L'invention est relative à une orthèse pour entorse externe de la cheville.

On connaît déjà, en particulier par le document US 4 628 945, des orthèses composées de coques en matière plastique venant de part et d'autre de la cheville et de la jambe en plaquant sur celle-ci des coussins, sous l'action de sangles entourant lesdites coques.

La présence de deux coques augmente l'encombrement général de la cheville et rend très désagréable le port d'une chaussure, même lorsqu'il s'agit d'une chaussure de sport, par exemple dite de tennis ou de basket. En effet, la souplesse des parois de ce type de chaussures ne permet pas d'absorber l'augmentation d'encombrement transversal de l'ensemble orthèse-cheville, de sorte que la cheville et la jambe sont comprimées en créant un sentiment d'inconfort.

En outre, la liaison de chacune des deux coques latérales avec une talonnette au moyen de sangles ou équivalents, ne procure pas à l'orthèse une rigidité suffisante lui permettant de limiter les mouvements d'inversion et d'éversion de la cheville.

On connaît par ailleurs des orthèses dénommées « releveurs de pied » destinées à brider une entorse du dessus du pied et dans lesquelles la coque externe est monolithique avec la coque interne de moindre hauteur et d'une plaque de soutien plantaire allant du talon à la voûte plantaire.

Aux inconvénients inhérents à l'encombrement de l'orthèse par rapport à la pointure des chaussures du patient, ce type d'orthèse ajoute, par son soutien plantaire, l'inconvénient de gêner la marche.

La présente invention a pour objet de fournir une orthèse remédiant à ces inconvénients et assurant donc un maintien de la cheville avec contrôle anti varus, tout en ne gênant pas les mouvements de flexion de cette cheville.

Elle concerne une orthèse comprenant une coque en matière plastique, apte à plaquer un coussin de confort contre la partie extérieure de la cheville et de la jambe, sous l'action de sangles de serrage.

Selon l'invention, la coque monolithique présente une forme en L comprenant un montant anatomique sur la face interne duquel est fixé le coussin de confort et une patte transversale apte à s'étendre sous le talon et sur laquelle sont fixées, d'une part, l'une des branches d'une sangle en Y et, d'autre part, une talonnette formée par un coussinet en matériau souple et antidérapant dont la face supérieure est pentue transversalement en allant du haut vers le bas, et de l'extérieur vers l'intérieur, ladite talonnette ne s'étendant que sous le talon, tant en largeur qu'en longueur.

Lorsque l'orthèse est posée et serrée sur la cheville, le montant de la coque s'étend verticalement contre la jambe, tandis que sa patte s'étend sous le talon et forme support pour la talonnette et pour la sangle en Y à laquelle elle est liée. Le monolithisme du montant et de la patte de la coque garantit la rigidité de l'ensemble et l'efficacité du maintien de la cheville, tandis que la limitation des dimensions de la patte et de la talonnette, pour qu'ils soient contenus sous le talon, favorise la marche.

Enfin, le recours a une seule coque, réduit l'encombrement de l'orthèse et n'entraîne pas d'inconfort lorsque le pied est engagé avec elle dans une chaussure.

Dans une forme d'exécution de l'invention, la sangle en Y est conformée de manière que ses deux branches libres, dont les extrémités sont munies de moyens de liaison complémentaires, tel que tronçon de ruban à crochets et tronçon de ruban à bouclettes, se superposent sur la face externe du montant de la coque et au-dessus de la malléole externe, en exerçant sur la jambe un effort d'appui coopérant avec la forme en coin de la talonnette pour assurer une fonction anti varus.

L'invention sera mieux comprise à l'aide de la description qui suit en référence au dessin schématique annexé représentant, à titre d'exemple, une forme d'exécution de l'orthèse selon l'invention.
Figure 1 est une vue en perspective de la coque de l'orthèse selon l'invention,
Figure 2 est une vue en perspective par-dessous de la coque et de ses composants avant assemblage avec ses composants,
Figure 3 est une vue de côté par l'extérieur d'une cheville équipée de l'orthèse selon l'invention,
Figure 4 est une vue par l'arrière d'une cheville gauche équipée de l'orthèse.

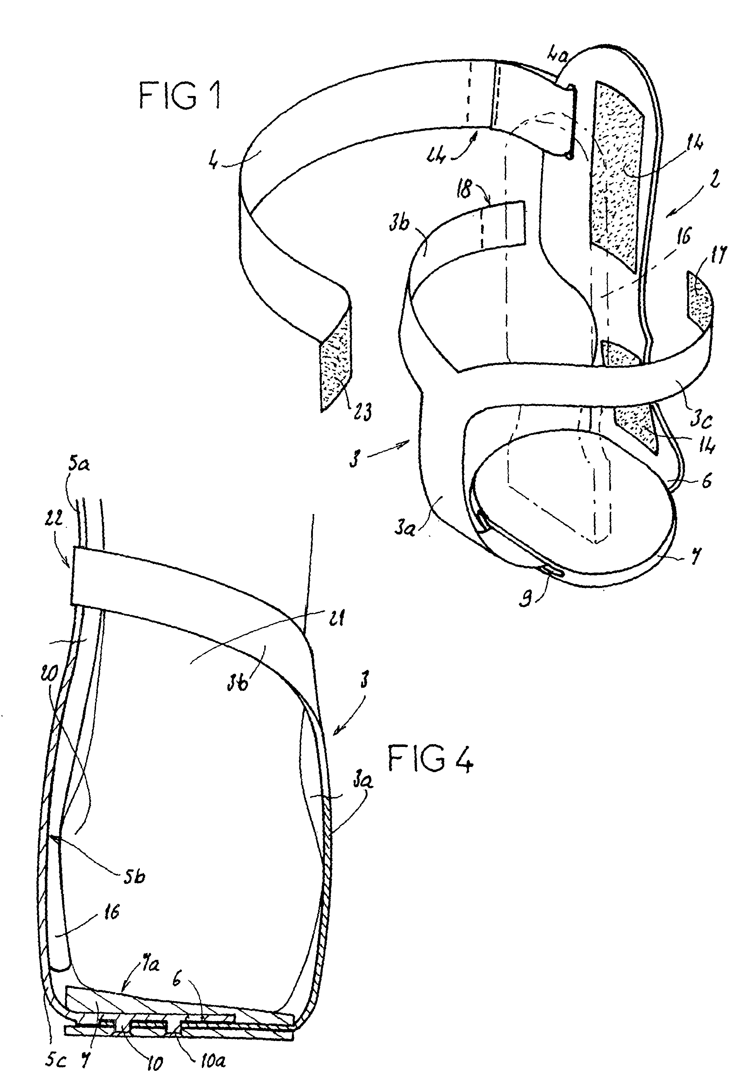

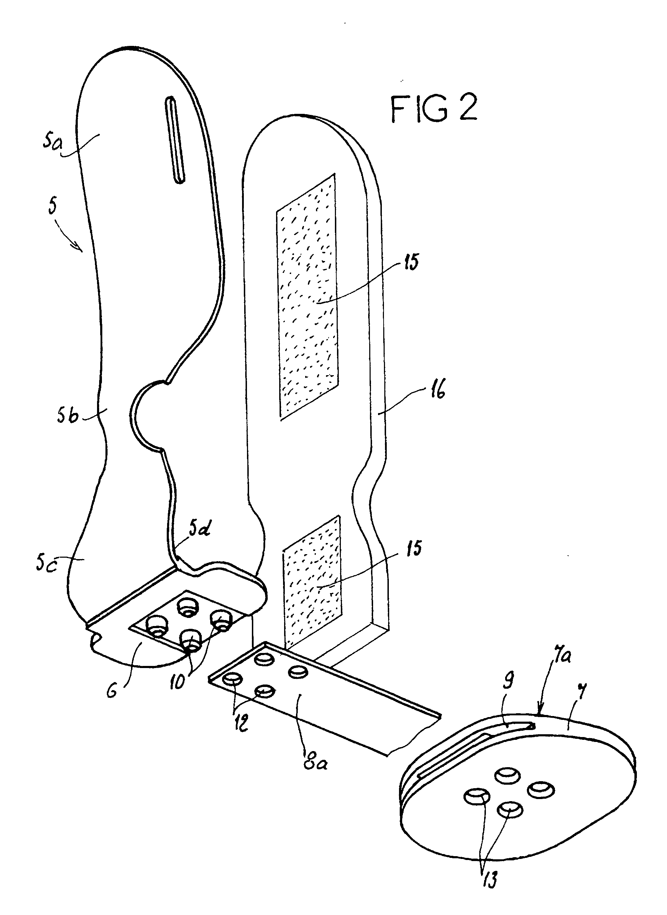

Comme montré en détails aux figures 1 et 2, l'orthèse est essentiellement composée d'une coque 2 en matière plastique semi-rigide et équipée de sangles 3 et 4. Cette coque est composée d'un montant anatomique 5 comportant une partie supérieure 5a, apte à être plaquée contre la jambe, une partie centrale coudée et échancrée 5b, apte à épouser la malléole externe de la cheville, et une partie inférieure 5c se raccordant, par une partie arrondie 5d, à une patte transversale 6. Cette patte est destinée à recevoir une talonnette 7 et à être reliée avec l'extrémité 8a de la branche 3a de la sangle 3, qui est en forme de Y.

Dans la forme d'exécution représentée, la talonnette 7, qui est constituée par un coussinet en matériau souple et antidérapant et par exemple en matière plastique, a des dimensions, transversale et longitudinale, telles qu'elle s'insère aisément sous le talon de la cheville, sans en déborder. Cette talonnette est traversée transversalement par une fente 9 grâce à laquelle elle peut être engagée sur l'extrémité 8a de la branche 3a de la sangle et sur la patte 6.

La figure 4 montre que la talonnette 7 présente une face supérieure 7a qui est pentue et qui va du haut vers le bas en allant de l'extérieur vers l'intérieur de la cheville.

Dans la forme d'exécution représentée, la liaison entre la patte 6, la branche 3a de la sangle 3 et la talonnette 7 est assurée par plusieurs doigts 10, en l'occurrence quatre, saillant de la face inférieure de la patte 6 et traversant, d'une part, des alésages 12 de mêmes dimension et répartition ménagés à l'extrémité 8a de la branche 3a, et d'autre part, des alésages 13 de plus grande dimension mais de même répartition, ménagés dans la paroi inférieure de la talonnette 7. Les alésages 13 sont destinés à accueillir la tête 10a qui est formée à l'extrémité de chacun des doigts 10 après fusion de l'extrémité de ceux-ci pour assurer la liaison mécanique des différents éléments 3a et 7 avec la patte 6.

La figure 4 montre que ce mode de liaison est peu encombrant et ne procure aucune gêne à l'utilisateur.

Comme montré figure 1, le montant 5 de la coque 2 comporte, sur sa face interne, plusieurs tronçons 14 de ruban à crochets ou à picots aptes à coopérer avec des tronçons 15 de ruban à bouclettes, montrés figure 2 et ménagés sur la face arrière d'un coussin de confort 16 et, par exemple, d'un coussin en matière plastique à cellules ouvertes, et élastiquement déformable.

La sangle en Y 3 est conformée de manière que ses deux branches supérieures 3b, 3c enveloppent la jambe au-dessus de la malléole externe 20, de manière que ses extrémités munies, l'une, d'un tronçon de ruban à crochets 17 et, l'autre, d'un tronçon de ruban à bouclettes 18, se superposent contre la face extérieure du montant 5 en favorisant le plaquage de ce montant sur la jambe 21, dans la zone d'appui représentée en 22 à la figure 4.

Près de son extrémité supérieure, le montant 5 de la coque 2 est lié à l'une des extrémités 4a de la sangle supérieure 4, sangle dont l'extrémité libre est munie d'un tronçon de ruban à crochets 23, apte à coopérer avec un tronçon de ruban à bouclettes 24 rapporté sur l'autre extrémité 4a de la sangle 4.

Lorsque cette orthèse est mise en place, sa patte 6 avec la talonnette 7 est disposée sous le talon de la cheville devant être maintenue, en prenant soin de disposer le montant 5 de la coque contre la partie extérieure de la cheville 1 et de la jambe 21. Il est d'abord procédé à l'entourage de la jambe 21 avec la sangle supérieure 4, puis à l'entourage de la partie intermédiaire au moyen des branches 3b, 3c de la sangle en Y 3, en prenant soin de communiquer à ces deux branches une tension telle que, par la liaison de leurs extrémités, elles plaquent le montant 5 de la coque 2 au-dessus de la cheville.

Dans cette position, et comme montré à la figure 4, le point d'appui 22, créé par les deux branches 3b, 3c sur la jambe 21 au-dessus de la malléole externe 20, coopère avec la face inclinée 7a de la talonnette 7 pour assurer une fonction anti varus. Ainsi, si la stabilisation est assurée par la coque externe en forme de L, chaque phase d'appui du talon sur le sol détend le ligament collatéral latéral (LLE) grâce à la forme antivarus de la talonnette. Outre un rôle antalgique cette talonnette évite toute récidive du traumatisme en inversion.

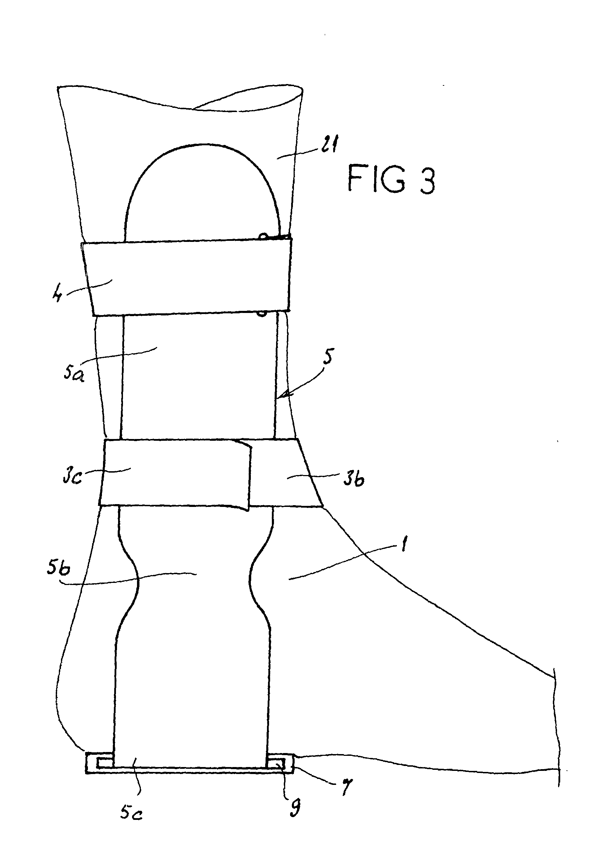

La figure 3 montre que la talonnette 7, qui ne dépasse pas du talon, ne gêne en rien les mouvements de flexion de la cheville, tandis que la figure 4 montre que, par sa structure à une seule coque, l'orthèse augmente peu l'encombrement transversal de la cheville qui, ainsi équipée, n'entraîne aucun inconfort lorsque le pied est passé dans une chaussure.

Une telle orthèse peut être utilisée :
- seule, pour le traitement des entorses externes moyennes,
- seule ou après plâtrage, pour le traitement des entorses externes graves ou pour la rééducation.

## Revendications

1. Orthèse pour entorse externe de la cheville comprenant une coque (2) en matière plastique, apte à plaquer un coussin de confort (16) contre la partie extérieure de la cheville et de la jambe, sous l'action de sangles de serrage (3, 4), **caractérisée en ce que** la coque est monolithique (2) et présente une forme en L comprenant un montant (5a,5b,5c) anatomique sur la face interne duquel est fixé le coussin de confort (16) et une patte transversale (6) apte à s'étendre sous le talon et sur laquelle sont fixées, d'une part, l'une des branches (3a) d'une sangle (3) en Y et, d'autre part, une talonnette(7) formée par un coussinet en matériau souple et antidérapant dont la face supérieure est pentue transversalement en allant du haut vers le bas, et de l'extérieur vers l'intérieur, ladite talonnette (7) ne s'étendant que sous le talon, tant en largeur qu'en longueur.

2. Orthèse selon la revendication 1, **caractérisée en ce que** la talonnette est traversée transversalement par une fente (9) par laquelle elle est engagée sur la patte transversale (6) de la coque (2) et sur l'extrémité correspondante (8a) de la sangle (3) en Y.

3. Orthèse selon l'ensemble des revendications 1 et 2, **caractérisée en ce que** la patte transversale (6) de la coque (2) est solidaire de plusieurs doigts (10) saillant vers le bas et aptes à pénétrer dans des alésages (12), ayant les mêmes dimensions et répartition qu'eux mais ménagés à l'extrémité de fixation (8a) de la sangle (3) en Y, et dans des alésages (13) de plus grande dimension mais de même répartition, ménagés dans la paroi inférieure de la talonnette (7), de manière que, après engagement de la talonnette (7) sur la patte (6) et fusion des extrémités des doigts (10), les têtes (10a) ainsi formées dans les alésages (13) de la talonnette assurent la liaison de l'extrémité de la sangle (3) avec la patte (6) de la coque et la retenue de cette talonnette (7).

4. Orthèse selon la revendication 1, **caractérisée en ce que** la sangle (3) en Y est conformée de manière que ses deux branches libres (3b, 3c), dont les extrémités sont munies de moyens de liaison complémentaires, tel que tronçon de ruban à crochets et tronçon de ruban à bouclettes, se superposent sur la face externe du montant (5) de la coque (2) et au-dessus de la malléole externe, en exerçant sur la jambe un effort d'appui coopérant avec la forme en coin de la talonnette (7) pour assurer une fonction anti varus.

## Patentansprüche

1. Orthese für ein extern verstauchtes Sprunggelenk mit einer Schale (2) aus Kunststoff, die unter der Wirkung von Festspannriemen (3, 4) ein Komfortkissen (16) gegen den Außenteil des Sprunggelenks und des Beins drücken kann, **dadurch gekennzeichnet, dass** die Schale (2) aus einem Stück besteht und eine L-Form mit einer anatomischen Stütze (5a, 5b, 5c), auf deren Innenseite das Komfortkissen (16) befestigt ist, und einem Queransatz (6) aufweist, der sich unter der Ferse erstrecken kann und an dem einerseits eine der Abzweigungen (3a) eines Y-förmigen Riemens (3) und andererseits eine durch ein Kissen aus flexiblem Material gebildete rutschfeste Ferseneinlage (7) befestigt ist, deren Oberseite in Querrichtung von oben nach unten und von außen nach innen verlaufend geneigt ist, wobei sich die Ferseneinlage (7) sowohl in der Breite als auch in der Länge nur unter der Ferse erstreckt.

2. Orthese nach Anspruch 1, **dadurch gekennzeichnet, dass** die Ferseneinlage in Querrichtung von einem Schlitz (9) durchquert wird, durch den sie mit dem Queransatz (6) der Schale (2) und dem entsprechenden Ende (8a) des Y-förmigen Riemens (3) in Eingriff steht.

3. Orthese nach den Ansprüchen 1 und 2, **dadurch gekennzeichnet, dass** der Queransatz (6) der Schale (2) fest mit mehreren nach unten vorragenden Fingern (10) verbunden ist, die in Bohrungen (12), welche die gleichen Abmessungen und die gleiche Verteilung wie sie aufweisen, aber am Befestigungsende (8a) des Y-förmigen Riemens (3) ausgebildet sind, und in in der Innenwand der Ferseneinlage (7) ausgebildete Bohrungen (13) größerer Abmessung, aber gleicher Verteilung eintreten können, so dass nach dem Eingriff der Ferseneinlage (7) mit dem Ansatz (6) und Schmelzen der Enden der Finger (10), die so gebildeten Köpfe (10a) in den Bohrungen (13) der Ferseneinlage die Verbindung des Endes des Riemens (3) mit dem Ansatz (6) der Schale und das Festhalten dieser Ferseneinlage (7) gewährleisten.

4. Orthese nach Anspruch 1, **dadurch gekennzeichnet, dass** der Y-förmige Riemen (3) so konfiguriert ist, dass seine beiden freien Abzweigungen (3b, 3c), deren Enden mit komplementären Verbindungsmitteln, wie zum Beispiel einem Haken- und einem Schlingenbandabschnitt, versehen sind, sich auf der Außenseite der Stütze (5) der Schale (2) und über dem Außenknöchel überlagern, indem sie auf das Bein eine Stützkraft ausüben, die mit der Keilform der Ferseneinlage (7) zusammenwirkt, um eine Anti-Varus-Funktion zu gewährleisten.

## Claims

1. Orthosis for an externally sprained ankle, comprising a plastic shell (2) which is able to press a comfort-enhancing liner (16) against the external aspect of the ankle and of the leg under the action of tightening straps (3, 4), **characterized in that** the shell (2) is made in one piece and has an L shape comprising an anatomical upright (5a, 5b, 5c), on the internal face of which the comfort-enhancing liner (16) is fixed, and a transverse tab (6) which is able to extend under the heel and on which are fixed, on the one hand, one of the branches (3a) of a Y-shaped strap (3), and, on the other hand, a heel piece (7) formed by a pad of supple and non-slip material whose upper face slopes transversely downwards, and from the outside inwards, said heel piece (7) extending only under the heel both in width and in length.

2. Orthosis according to Claim 1, **characterized in that** the heel piece is traversed transversely by a slot (9) via which it is engaged on the transverse tab (6) of the shell (2) and on the corresponding end (8a) of the Y-shaped strap (3).

3. Orthosis according to both of Claims 1 and 2, **characterized in that** the transverse tab (6) of the shell (2) is made integral with several fingers (10) which project downwards and are able to penetrate into bores (12) having the same dimensions and distribution as them, but being formed at the securing end (8a) of the Y-shaped strap (3), and also into bores (13) of greater dimension but same distribution formed in the lower wall of the heel piece (7) in such a way that, after engagement of the heel piece (7) on the tab (6) and melting of the ends of the fingers (10), the heads (10a) thus formed in the bores (13) of the heel piece ensure connection of the end of the strap (3) to the tab (6) of the shell and retention of this heel piece (7).

4. Orthosis according to Claim 1, **characterized in that** the Y-shaped strap (3) is configured in such a way that its two free branches (3b, 3c), whose ends are provided with complementary connection means such as a tape section with hooks and a tape section with loops, are superposed on one another on the outer face of the upright (5) of the shell (2) and above the external malleolus, exerting on the leg a pressing force which cooperates with the wedge shape of the heel piece (7) to prevent varus.
